# EUROPÄISCHE PATENTSCHRIFT

(11) **EP 2 628 500 B1**
(45) Veröffentlichungstag und Bekanntmachung des Hinweises auf die Patenterteilung: **28.05.2014**
(21) Anmeldenummer: 12001013.7
(22) Anmeldetag: 16.02.2012
(51) Int. Cl.: A61M 27/00, A61M 1/00

(54) **Wunderversorgungsanordnung**
Wound treatment assembly
Agencement de traitement des plaies

(43) Veröffentlichungstag der Anmeldung: 21.08.2013
(73) Patentinhaber: Lohmann & Rauscher GmbH, 2525 Schönau/Triesting (AT)
(72) Erfinder: Danei, Federico, 1180 Wien (AT); Wagner, Georg, 4040 Linz (AT); Rohrer, Christian, 9500 Villach (AT); Steinlechner, Erik, 2500 Baden (AT)
(74) Vertreter: Leinweber & Zimmermann

(56) Entgegenhaltungen:
- WO-A1-2005/046760
- WO-A1-2008/008032
- DE-C- 847 475
- US-A1- 2010 049 166

## Beschreibung

Die Erfindung betrifft eine Wundversorgungsanordnung mit zwei Drainageschichten, denen eine andererseits mit einer Absaugeinrichtung verbindbare Sauganordnung zum Absaugen von zwischen die Drainageschichten gelangendem Exsudat zugeordnet ist.

Derartige Wundversorgungsanordnungen sind in der EP 1 674 127 A1 beschrieben. Sie werden beispielsweise zum Entfernen von Wundsekreten aus Körperhöhlen benutzt. In der genannten Schrift werden Anordnungen zum Entfernen von Wundsekreten aus Körperhöhlen in Form von Drainageschläuchen, Bauchtüchern und Schwämmen als bekannt vorausgesetzt. Dabei wird es als problematisch beanstandet, daß Drainageschläuche sehr schnell zur Bildung von Kanälen in der Körperhöhle führen, so daß das Wundsekret nicht mehr vollständig abgesaugt wird, der Austausch von Bauchtüchern mit einem großen chirurgischen Aufwand verbunden ist und Schwämme schnell zur Verstopfung neigen, so daß ein gegebenenfalls im Bereich des Schwamms aufgebauter Unterdruck nicht mehr gleichmäßig über die Wundoberfläche verteilt werden kann.

Zur Lösung dieser Probleme wird in der genannten Schrift eine Wundversorgungsanordnung der eingangs beschriebenen Art vorgeschlagen, bei der die Drainageschichten einen offenporigen Schaumstoffkörper aus Polyurethan mit einer Porengröße von 10-75 ppi (Pores per Inch) und einer Rohdichte von 14-75 kg/m³ aufweisen und bei der mindestens ein Absaugschlauch mit zwei offenen Enden vorgesehen ist, dessen eines offenes Ende zwischen zwei benachbarten Drainageschichten eingebracht ist, wobei sich das zweite offene Ende des Schlauches außerhalb der Drainageschichten befindet.

Der im Rahmen dieser Erfindung als Sauganordnung anzusprechende Schlauch kann bei der bekannten Anordnung neben dem offenen Ende weitere Öffnungen aufweisen, die im Mantel des Schlauchs ausgebildet sind. Dabei sind die weiteren Öffnungen gleichmäßig über den Teil des Schlauchs, der sich zwischen den Drainageschichten befindet, verteilt. Die Öffnungen können in Abhängigkeit von dem Schlauchaußendurchmesser 1-15 mm groß sein. Bevorzugt gleicht die Größe der Öffnungen der Größe des offenen Endes des Schlauchs, der zwischen den Drainageschichten angeordnet ist. Alternativ kann vorgesehen sein, daß die Größen der weiteren Öffnungen mit zunehmender Entfernung von dem offenen Ende des zwischen den Drainageschichten angeordneten Schlauchabschnitts zunehmen. Der Offenbarungsgehalt der EP 1 674 127 wird hinsichtlich der Art des Schlauchs und der darin vorgesehenen Öffnungen hiermit durch ausdrückliche Inbezugnahme in diese Beschreibung mit aufgenommen.

Beim Einsatz der in der EP 1 674 127 A1 beschriebenen Wundversorgungsanordnungen hat sich allerdings gezeigt, daß weiterhin unbefriedigende Absaugergebnisse erreicht werden, die auf Verstopfungen des Schlauchs oder der Drainageschichten zurückzuführen sind.

Zur Lösung dieser Probleme wird in der DE 20 2009 005 780 U1 eine Vorrichtung zum Entfernen von Wundsekreten vorgeschlagen, die zwei aufeinanderliegende, elastische, poröse, weiche, Flüssigkeit leitende Labyrinthmateriallagen aufweist, welche das Wundsekret aus der Körperhöhle durch einen Abflußschlauch ableiten. Allerdings hat es sich auch bei Einsatz dieser Vorrichtungen gezeigt, daß nur eine unbefriedigende Entfernung des Wundsekrets bzw. Exsudats aus der Körperhöhle erreicht wird, wobei das Exsudatmanagement mit zunehmender Einsatzdauer der bekannten Vorrichtungen problematischer wird.

Wundversorgungsanordnungen nach dem Oberbegriff des Patentanspruchs 1 sind in der WO 2008/008032 A1 und der US 2010/0049166 A1 beschrieben.

Angesichts der vorstehend beschriebenen Probleme im Stand der Technik liegt der Erfindung die Aufgabe zugrunde, eine Wundversorgungsanordnung zum Absaugen von Wundexsudat aus Körperhöhlen im Rahmen der Unterdrucktherapie bereitzustellen, mit der ein zufriedenstellendes Exsudatmanagement auch über einen längeren Zeitraum möglich ist.

Erfindungsgemäß wird diese Aufgabe durch die im kennzeichnenden Teil des Patentanspruchs 1 angegebene Weiterbildung der bekannten Wundversorgungsanordnungen gelöst.

Die Erfindung geht dabei auf die folgende Erkenntnis zurück:
Körperhöhlen, in die Drainagen eingeführt werden, sind regelmäßig luftdicht abgedeckt, um einen Unterdruck im Bereich der Körperhöhle erzeugen zu können. Nach Verbindung der Drainage mit der Pumpe wird eine Luft-Exsudat-Mischung aus der Körperhöhle abgesaugt. Sobald das ganze durch die Saugwirkung aus der Körperhöhle lösbare Exsudat abgesaugt ist, fällt die Luft-Exsudat-Strömungsdichte im Schlauch ab. Das in dem Schlauch enthaltene Exsudat wird nicht weiter transportiert und es kann damit zu einer Koagulation des Exsudats und damit auch zu einer Verstopfung des Schlauchs kommen. Dieses Risiko wird auch nicht durch die in der DE 20 2009 005 780 vorgeschlagenen Labyrinthmateriallagen beseitigt, weil es auch außerhalb der Labyrinthmateriallagen entsteht.

Erfindungsgemäß ist der Sauganordnung eine Spülanordnung zum Zuführen zumindest eines Fluids, wie etwa einer Flüssigkeit oder eines gasförmigen Fluids, wie etwa Luft, zwischen die Drainageschichten zugeordnet. Durch die Spülanordnung strömt das Fluid in die Körperhöhle ein. Dadurch wird kontinuierlich der Fluß in der Sauganordnung aufrechterhalten. Koagulationen werden vermieden, weil Wundexsudat kontinuierlich durch die Absauganordnung in Richtung auf die Absaugeinrichtung abgesaugt wird.

Im Rahmen der Erfindung hat es sich weiter überraschenderweise gezeigt, daß bei der Vakuumtherapie, bei der erfindungsgemäße Wundversorgungsanordnungen regelmäβig eingesetzt werden, ein gleichzeitig mit dem Erzeugen des Unterdrucks erfolgender kontinuierlicher Fluideintritt in den Wundbereich den Heilungsvorgang weiter verbessert. Ein durch die Spülanordnung möglicher kontinuierlicher Fluideintritt in die Körperhöhle verursacht, nach dem Verbinden einer Absaugeinrichtung mit der Sauganordnung, einen kontrollierten und kontinuierlichen Druckabfall in dem Wundbereich, der durch das Absaugen kontinuierlich von der Sauganordnung ausgeglichen werden muss. Dadurch kann die Entfernung des Exsudats und damit auch der Heilungsvorgang weiter verbessert werden.

Wie der vorstehenden Erläuterung aus der EP 1 674 127 A1 bekannter Wundversorgungsanordnungen zu entnehmen ist, kann die Sauganordnung mindestens einen Absaugschlauch mit einem zwischen den Drainageschichten angeordneten, ggf. verzweigten Saugabschnitt und einem außerhalb der Drainageschichten freiliegenden und mit der Absaugeinrichtung verbindbaren Leitungsabschnitt aufweisen, wobei der Saugabschnitt neben der Schlauchöffnung auch ein, zwei, drei oder mehr weitere Öffnungen aufweisen kann, welche längs der Schlauchachse hintereinander und/oder längs einer die Schlauchachse wendelförmig umlaufenden Linie angeordnet sein können. Hinsichtlich der Größe und Anordnung der in dem Saugabschnitt vorgesehenen und zum Einleiten des Exsudats in das vom Schlauchmantel umlaufene Schlauchvolumen dienenden Öffnungen wird Bezug genommen auf den Offenbarungsgehalt der EP 1 674 127 A1, welcher hiermit durch ausdrückliche Inbezugnahme in diese Beschreibung aufgenommen wird. Es ist nicht ausgeschlossen, daß der Saugabschnitt und der Leitungsabschnitt des Absaugschlauchs zwei verschiedenen Formen aufweisen kann. Insbesondere kann der Saugabschnitt einen rechteckigen Querschnitt aufweisen, wobei der Leitungsabschnitt einen runden Querschnitt aufweist.

Die Sauganordnung kann aus einem der marktüblichen thermoplastischen oder elastomerischen Materialien für medizinische Schläuche hergestellt werden. Insbesondere kann die Sauganordnung aus Silikon, Polyurethan (PU) oder Polyvinylchlorid (PVC) hergestellt werden. Der Saugabschnitt der Sauganordnung kann röntgenkontrastfähig sein, um die korrekte Positionierung der Wundversorgungsanordnung, unter Röntgen, bestimmen zu können. Der Schlauch kann flexibel und elastisch sein, um eine Anpassung der Wundversorgungsanordnung an die Form der Körperhöhle zu ermöglichen, ist zweckmäßigerweise aber auch starr genug, um unter dem angewandten Unterdruck nicht zu kollabieren.

Zusätzlich zu einem Schlauch gemäß der EP 1 674 127 kann eine Absauganordnung einer erfindungsgemäßen Wundversorgungsanordnung auch einen Sammelraum am Rand der Drainageschichten aufweisen, aus dem das Exsudat dann mit einem geeigneten Schlauch abgesaugt wird. Diesbezüglich wird Bezug genommen auf den Offenbarungsgehalt der DE 20 2009 005 780 U1, deren Offenbarungsgehalt hinsichtlich der Ausbildung des Sammelraums hiermit durch ausdrückliche Inbezugnahme in diese Beschreibung mit aufgenommen wird.

Ähnlich wie die Sauganordnung kann auch die Spülanordnung einer erfindungsgemäßen Wundversorgungsanordnung einen Spülschlauch mit einem zwischen die Drainageschichten mündenden, vorzugsweise zwischen den Drainageschichten angeordneten Spülabschnitt und einem außerhalb der Drainageschichten freiliegenden Zuführabschnitt aufweisen. Über den Spülschlauch können flüssige und/oder gasförmige Fluide in die Körperhöhle eingeleitet werden, um so einen kontinuierlichen Strom in der Sauganordnung bereitzustellen.

Bei einer besonders bevorzugten Ausführungsform der Erfindung sind Sauganordnung und Spülanordnung in einem gemeinsamen Multilumenschlauch integriert, dessen eines Lumen an die Absaugeinrichtung angeschlossen ist und dessen anderes Lumen zum Zuführen eines Fluids in die Körperhöhle dient. Der Multilumenschlauch kann zwei, drei oder mehr Lumen aufweisen, wobei zusätzlich zu dem die Sauganordnung verwirklichenden Lumen, dem die Spülanordnung verwirklichenden Lumen auch noch ein Lumen zum Zuführen einer Wirksubstanz in die Körperhöhle vorgesehen sein kann.

Die Spülanordnung, ähnlich wie die Sauganordnung, kann aus einem der marktüblichen thermoplastischen oder elastomerischen Materialien für medizinische Schläuche hergestellt werden. Insbesondere kann die Spülanordnung aus Silikon, Polyurethan (PU) oder Polyvinylchlorid (PVC) hergestellt werden. Die Spülanordnung kann flexibel und elastisch sein, um eine Anpassung der Wundversorgungsanordnung an die Form der Körperhöhle zu ermöglichen, ist zweckmäßigerweise aber auch starr genug, um unter dem angewandten Unterdruck nicht zu kollabieren.

Die Sauganordnung und die Spülanordnung können aus demselben Material oder aus zwei verschiedenen Materialien hergestellt werden.

Im Rahmen der Erfindung hat es sich als besonders wirkungsvoll erwiesen, wenn zwischen den Drainageschichten auch noch eine offenporige, vorzugsweise Gaze aufweisende, Saugschicht angeordnet ist, in der das durch die Drainageschicht gelangende Exsudat gesammelt wird und aus dem das Exsudat mit Hilfe der Sauganordnung abgesaugt werden kann. Die Gaze kann aus 100% Baumwolle bestehen, oder aus einer Mischung von Baumwollfasern und synthetischen Fasern, oder aus 100% synthetischen Fasern. Die Gaze ist vorzugsweise hydrophil, um einen Austritt des in der Saugschicht gesammelten Exsudats am Ende der Therapie (beim Entfernen der Wundversorgungsanordnung aus der Körperhöhle) zu vermeiden. Die Gaze kann auch einen oder mehrere Röntgenkontrastfäden enthalten, um die korrekte Positionierung der Wundversorgungseinrichtung in der Körperhöhle unter Röntgen zu bestimmen. Eine wesentliche Eigenschaft der Drainageschicht ist, daß sie eine dreidimensionale offenporige Struktur aufweist, die unter dem angewandten Unterdruck eine nicht zu 100% (vollständig) komprimierte (und damit noch porenhaltige) Struktur ergibt. Die Drainageschicht ist zweckmäßigerweise auch flexibel und weich genug, um eine Anpassung der Wundversorgungsanordnung an die Körperhöhle, in die sie eingebracht ist, zu ermöglichen.

In einer besonderen Ausführung der Erfindung, kann die Saugschicht eine antimikrobielle Wirkung aufweisen. In einer weiteren Ausführung der Erfindung, weisen die Drainageschichten eine antimikrobielle Beschichtung auf. Die Drainageschichten einer erfindungsgemäßen Wundversorgungsanordnung sind vorzugsweise etwa flächengleich und miteinander verbunden, insbesondere vernäht und/oder verklebt und/oder verschweißt, um so eine zusammenhängende Baugruppe bereitzustellen. Die Verbindung zwischen den Drainageschichten findet in konzentrischen Linien statt. Die äußerste Verbindung findet sich längs der umlaufenden Ränder der Drainageschichten, die innerste Verbindung längs der umlaufenden Ränder des Saugabschnitts der Sauganordnung oder, falls vorhanden, der Saugschicht. Die innerste Verbindung dient als Arretierungshilfe für die Sauganordnung bzw. Saugschicht zwischen den Drainageschichten. Zwischen der äußersten und der innersten Verbindung können noch eine, zwei oder mehrere Zwischenverbindungen vorhanden sein, so daß der Anwender durch Schneiden die Größe der Wundversorgungsanordnung an die Größe der Körperhöhle anpassen kann.

Zur Vermeidung von Wundinfektionen ist der zum Einleiten des Fluids in die Körperhöhle dienenden Spülanordnung zweckmäßigerweise ein steriles Filter zugeordnet. Das sterile Filter weist eine Porengröße und eine Dicke auf, die eine Bakterienrückhalterate von mindestens 95% gewährleistet. Das sterile Filter kann hydrophob oder hydrophil sein, je nachdem ob das Spülfluid ein gasförmiges Fluid oder ein flüssiges Fluid ist. Das Filter kann z.B. aus Polytetrafluoräthylen (PTFE), Polyethylen (PE), Polypropylen (PP), Polyvinylidenfluorid (PVDF), Polyethersulfon (PES), Methyl Cellulose (MCE), Acrylic Copolymer oder Nylon hergestellt werden.

Das sterile Filter ist mit dem offenen Ende der Spülanordnung verbunden. Das Filter kann direkt am offenen Ende der Spülanordnung oder in einem Schlauchadapter angeordnet werden. Der Schlauchadapter wird in diesem Fall mit dem offenen Ende der Spülanordnung verbunden.

Eine wesentliche Eigenschaft des sterilen Filters ist, daß das Filter einen für die Anwendung passenden Fluidstrom ermöglicht. Der Fluidstrom soll nicht zu gering sein, um die kontinuierliche Absaugung der Pumpe nicht zu beeinträchtigen, und nicht zu stark, um die Erreichung des gewünschten Unterdrucks in dem Wundbereich zu ermöglichen.

Die Drainageschichten sind erfindungsgemäß entsprechend den Wundabdeckungen gemäß der DE 10 2009 019 646 A1 ausgeführt. Der Offenbarungsgehalt dieser Schrift wird hinsichtlich der Struktur der Drainageschichten bzw. Wundabdeckungen hiermit durch ausdrückliche Inbezugnahme in diese Beschreibung mit aufgenommen. Die Drainageschichten weisen demnach zweckmäßigerweise zwei parallel zueinander verlaufende, bahnförmige Elemente auf, zwischen denen ein Drainageraum gebildet ist, dessen Tiefe in einer sich etwa senkrecht zu den bahnförmigen Elementen erstreckenden Tiefenrichtung eine Kapillarwirkung auf in dem Drainageraum aufgenommene Exsudate gewährleistet. Die Tiefe des Drainageraums kann dazu 5 mm oder weniger betragen. Sie beträgt vorzugsweise aber 0,5 mm oder mehr. Dabei weist jedes der bahnförmigen Elemente zweckmäßigerweise einen den Durchtritt von Körperflüssigkeit in den Drainageraum ermöglichende Öffnung auf, wobei mindestens eine Öffnung durch einen sich ausgehend von einem der bahnförmigen Elemente in Richtung auf die gegenüberliegende innere Begrenzungsfläche des anderen bahnförmigen Elements erstreckenden und in den Drainageraum mündenden Kanal gebildet ist, dessen Kanalwand einstückig mit dem bahnförmigen Element ausgeführt, insbesondere durch Perforation des bahnförmigen Elements gebildet ist. Hinsichtlich weiterer Einzelheiten der vorzugsweise einzusetzenden Drainageschichten wird auf die DE 10 2009 019 646 A1 verwiesen.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich Bezug genommen wird, erläutert. In der Zeichnung zeigt:
- Fig. 1: eine Draufsicht auf eine erfindungsgemäße Wundversorgungsanordnung gemäß einer ersten Ausführungsform der Erfindung,
- Fig. 2: eine Draufsicht auf eine erfindungsgemäße Wundversorgungsanordnung gemäß einer zweiten Ausführungsform der Erfindung,
- Fig. 3: eine Darstellung der Wundversorgungsanordnung gemäß Fig. 1 ohne obere Drainageschicht,
- Fig. 4: eine Draufsicht auf die Wundversorgungsanordnung gemäß Fig. 1 mit schematisch angedeuteten Verbindungslinien,
- Fig. 5: eine schematische Schnittdarstellung der Wundversorgungsanordnung gemäß Fig. 1 mit (oben) und ohne (unten) Saugschicht und
- Fig. 6: im Rahmen der Erfindung einsetzbare Saug- und Spülanordnungen.

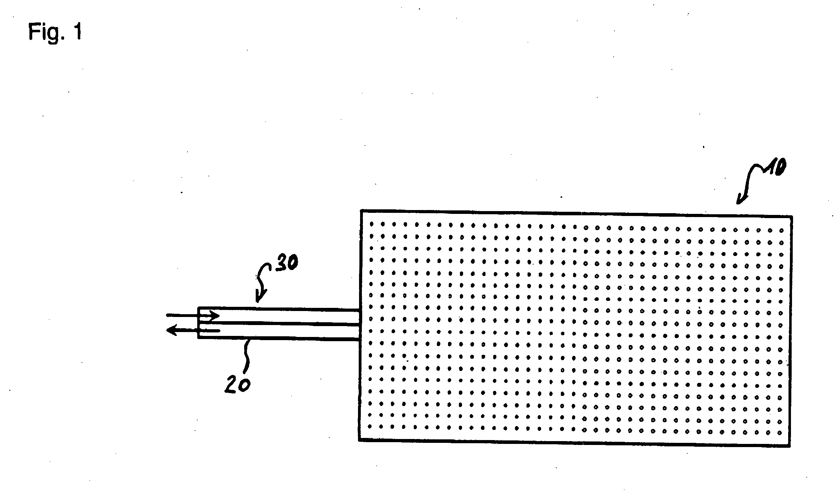

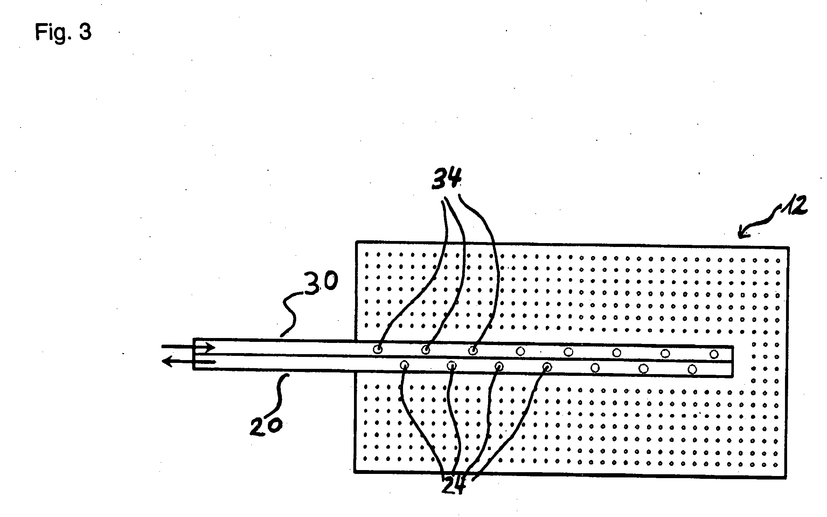

Die in Fig. 1 dargestellte Wundversorgungsanordnung umfaßt zwei Drainageschichten (10, 12), von denen in Fig. 1 nur die obere Drainageschicht 10 erkennbar ist, eine Sauganordnung 20 und eine Spülanordnung 30. Sauganordnung 20 und Spülanordnung 30 sind jeweils in Form von Schläuchen gebildet, wobei von dem Absaugschlauch 20 nur ein Leitungsabschnitt und von dem Spülschlauch 30 nur ein Zuführabschnitt erkennbar ist. Die Saug- und Spülabschnitte der Sauganordnung 20 bzw. Spülanordnung 30 sind zwischen den Drainageschichten (10, 12) angeordnet, wie eine vergleichende Betrachtung der Fig. 1 und 3 ergibt.

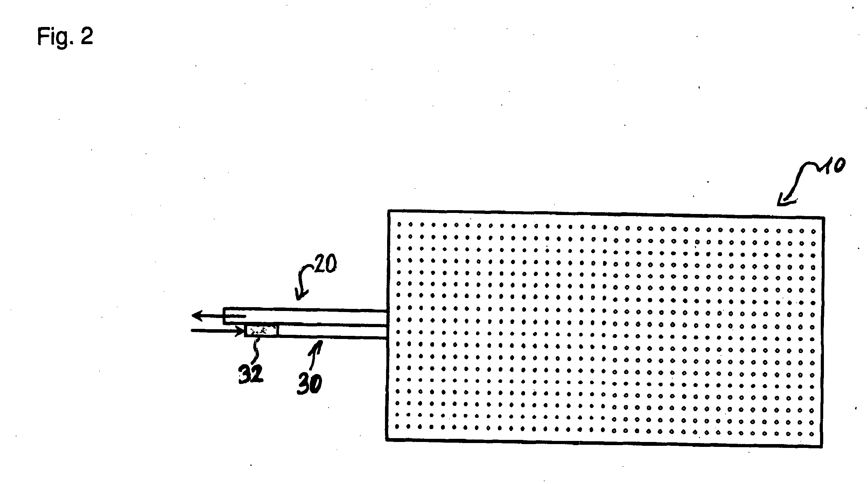

Die in Fig. 2 dargestellte Ausführungsform der Erfindung unterscheidet sich lediglich dadurch von der anhand der Fig. 1 erläuterten Ausführungsform, daß der Spülanordnung 30 ein antibakterielles Filter 32 zugeordnet ist, mit dem einerseits ein für die gewünschte Anwendung geeigneter Fluidstrom sichergestellt werden kann und andererseits eine schädliche Kontamination der Wunde verhindert wird.

Bei der Darstellung der erfindungsgemäßen Wundversorgungsanordnung gemäß Fig. 3 ist die obere Drainageschicht 10 abgenommen. Erkennbar ist daher nur noch die untere Drainageschicht 12. Eine vergleichende Betrachtung der Fig. 1 und 3 ergibt, daß die Sauganordnung 20 und die Spülanordnung 30 mit ihrem jeweiligen Saugabschnitt und Spülabschnitt zwischen der unteren Drainageschicht 12 und der oberen Drainageschicht 10 angeordnet sind. Zusätzlich zu den stirnseitigen Öffnungen weisen sowohl der Saugabschnitt als auch der Spülabschnitt Wandöffnungen 24 und 34 auf, um einerseits eine zufriedenstellende Absaugung und andererseits eine ausreichende Spülung zu erreichen.

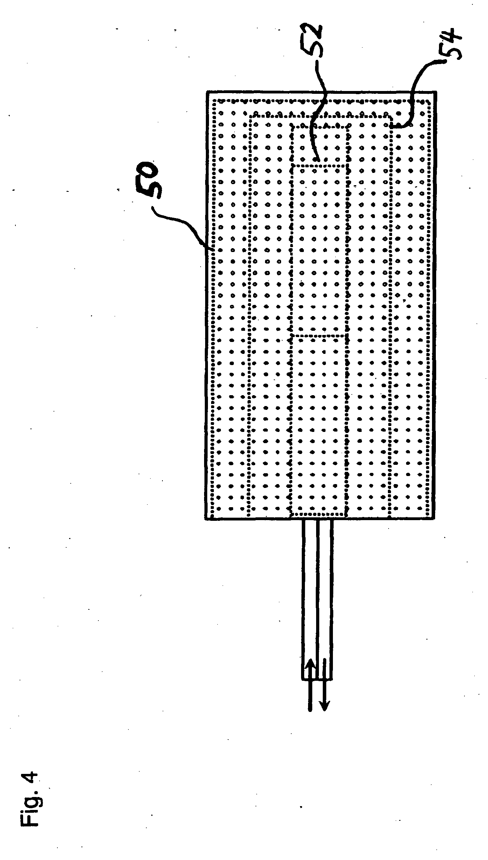

Gemäß Fig. 4 sind die obere Drainageschicht 10 und die untere Drainageschicht 12 längs Verbindungslinien 50, 52 und 54 miteinander verbunden, bspw. vernäht, verklebt oder verschweißt, insbes. ultraschallverschweißt.

Die innere Verbindungslinie 52 begrenzt eine Kammer zur Aufnahme des Saugabschnitts und des Spülabschnitts der Sauganordnung 20 und der Spülanordnung 30.

Die Bereitstellung einer äußeren Verbindungslinie 50 und einer mittleren Verbindungslinie 54 ermöglicht einen Zuschnitt der Größe der Wundversorgungsanordnung in Abhängigkeit von der zu versorgenden Wunde.

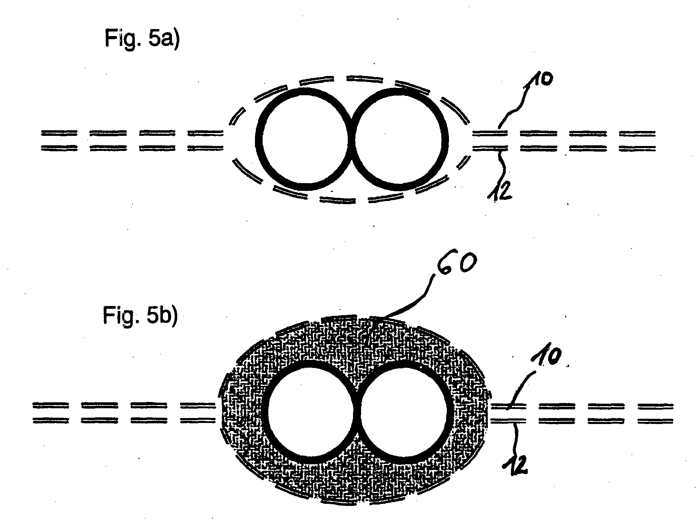

Gemäß Fig. 5a) sind Saugabschnitt und Spülabschnitt unmittelbar zwischen den Drainageschichten 10 und 12 angeordnet.

Bei der Anordnung gemäß Fig. 5b) ist zwischen Saugabschnitt und Spülabschnitt einerseits und Drainageschichten 10, 12 andererseits eine bspw. in Form von Gaze verwirklichte Saugschicht 60 angeordnet.

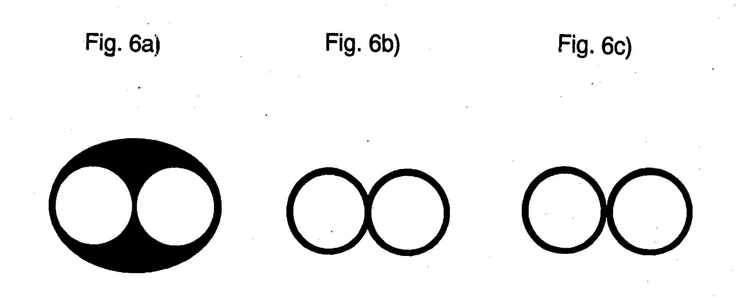

Gemäß Fig. 6a) können Sauganordnung 20 und Spülanordnung 30 als doppellumiger Schlauch mit einfachem Schlauchmantel ausgeführt sein.

Gemäß Fig. 6b) können Sauganordnung 20 und Spülanordnung 30 als doppellumiger Schlauch mit "8-Form" ausgeführt sein.

Gemäß Fig. 6c) können Sauganordnung 20 und Spülanordnung 30 in Form von zwei parallelen Schläuchen ausgeführt sein, die miteinander verbunden, insbes. verklebt oder mit Gummiringen zusammengehalten, sind.

Die Erfindung ist nicht auf die anhand der Zeichnung erläuterten Ausführungsbeispiele beschränkt. Vielmehr ist auch an Ausführungsformen gedacht, bei denen sich die Saugschicht vollflächig zwischen den Drainageschichten (10, 12) erstreckt. Auch kann zusätzlich zu der Sauganordnung 20 und der Spülanordnung 30 ein drittes Schlauchlumen vorgesehen sein, um bspw. einen Wirkstoff zuzuführen.

### BEZUGSZEICHENLISTE

- 10: obere Drainageschicht
- 12: untere Drainageschicht
- 20: Sauganordnung / Absaugschlauch
- 24: Wandöffnungen
- 30: Spülanordnung / Spülschlauch
- 32: antibakterielles Filter
- 34: Wandöffnungen
- 50, 52, 54: Verbindungslinien
- 60: Saugschicht

## Patentansprüche

1. Wundversorgungsanordnung mit zwei Drainageschichten (10, 12), denen eine andererseits mit einer Absaugeinrichtung verbindbare Sauganordnung (20) zum Absaugen von zwischen die Drainageschichten (10, 12) gelangendem Exsudat zugeordnet ist, wobei der Sauganordnung (20) eine Spülanordnung (30) zum Zuführen eines Fluids zwischen die Drainageschichten (10, 12) zugeordnet ist, **dadurch gekennzeichnet, daß** mindestens eine Drainageschicht zwei etwa parallel zueinander verlaufende bahnförmige Elemente aufweist, zwischen denen ein Drainageraum gebildet ist, dessen Tiefe in einer senkrecht zu den bahnförmigen Elementen erstreckenden Tiefenrichtung eine Kapillarwirkung auf in dem Drainageraum aufgenommene Exsudate gewährleistet.

2. Wundversorgungsanordnung nach Anspruch 1, **dadurch gekennzeichnet, daß** die Sauganordnung (20) mindestens einen Absaugschlauch mit einem zwischen den Drainageschichten (10, 12) angeordneten, ggf. verzweigten Saugabschnitt und einem außerhalb der Drainageschichten (10, 12) freiliegenden und mit der Absaugeinrichtung verbindbaren Leitungsabschnitt aufweist.

3. Wundversorgungsanordnung nach Anspruch 2, **dadurch gekennzeichnet, daß** ein Schlauchmantelabschnitt des Saugabschnitts mindestens ein, vorzugsweise zwei, drei oder mehr Öffnungen zum Einleiten des Exsudats in das vom Schlauchmantel umlaufene Schlauchlumen aufweist.

4. Wundversorgungsanordnung nach einem der vorhergehenden Ansprüche, **dadurch gekennzeichnet, daß** die Spülanordnung (30) einen Spülschlauch mit einem zwischen die Drainageschichten (10, 12) mündenden, vorzugsweise zwischen den Drainageschichten (10, 12) angeordneten Spülabschnitt und einem außerhalb der Drainageschichten (10, 12) freiliegenden Zuführabschnitt aufweist.

5. Wundversorgungsanordnung nach einem der vorhergehenden Ansprüche, **dadurch gekennzeichnet, daß** Sauganordnung (20) und Spülanordnung (30) einen Multilumenschlauch aufweisen.

6. Wundversorgungsanordnung nach einem der vorhergehenden Ansprüche, **gekennzeichnet durch** eine zwischen den Drainageschichten (10, 12) angeordnete, vorzugsweise Gaze aufweisende Saugschicht.

7. Wundversorgungsanordnung nach einem der vorhergehenden Ansprüche, **dadurch gekennzeichnet, daß** die Drainageschichten (10, 12) etwa flächengleich und längs ihrer umlaufenden Ränder zumindest abschnittweise miteinander verbunden, insbesondere vernäht und/oder verklebt sind.

8. Wundversorgungsanordnung nach einem der vorhergehenden Ansprüche, **dadurch gekennzeichnet, daß** der Spülanordnung (30) ein antibakterielles Filter (32) zugeordnet ist.

9. Wundversorgungsanordnung nach einem der vorhergehenden Ansprüche, **dadurch gekennzeichnet, daß** die Tiefe des Drainageraums 5 mm oder weniger und/oder mindestens 0,5 mm beträgt.

10. Wundversorgungsanordnung nach einem der vorhergehenden Ansprüche, **gekennzeichnet durch** eine an der die zu versorgende Wunde umgebenden Haut luftdicht festlegbare und von dem Absaugschlauch (20) durchsetzte Abdeckeinrichtung, insbesondere Abdeckfolie.

## Claims

1. A wound treatment assembly having two drainage layers (10, 12) to which a suction arrangement (20) that can be connected on the other side to a suction device for suctioning exudate passing between the drainage layers (10, 12) is assigned, a rinsing arrangement (30) for supplying a fluid between the drainage layers (10, 12) being assigned to the suction arrangement (20), **characterised in that** at least one drainage layer has two web-shaped elements running approximately parallel to one another and between which a drainage space is formed, the depth of which in a depth direction extending perpendicularly to the web-shaped elements guarantees a capillary action on exudates received in the drainage space.

2. The wound treatment assembly according to Claim 1, **characterised in that** the suction arrangement (20) has at least one suction hose with a suction section, optionally branched, and disposed between the drainage layers (10, 12), and a line section exposed outside of the drainage layers (10, 12) and connectable to the suction device.

3. The wound treatment assembly according to Claim 2, **characterised in that** a hose jacket section of the suction section has at least one, preferably two, three or more openings for introducing the exudate into the hose lumen surrounded by the hose jacket.

4. The wound treatment assembly according to any of the preceding claims, **characterised in that** the rinsing arrangement (30) has a rinsing hose with a rinsing section opening out between the drainage layers (10, 12), preferably disposed between the drainage layers (10, 12) and a supply section exposed outside of the drainage layers (10, 12).

5. The wound treatment assembly according to any of the preceding claims, **characterised in that** the suction arrangement (20) and the rinsing arrangement (30) have a multi-lumen hose.

6. The wound treatment assembly according to any of the preceding claims, **characterised by** a suction layer disposed between the drainage layers (10, 12) and preferably featuring gauze.

7. The wound treatment assembly according to any of the preceding claims, **characterised in that** the drainage layers (10, 12) are approximately coextensive and are connected to one another, at least in sections, along their peripheral edges, in particular sewn and/or glued.

8. The wound treatment assembly according to any of the preceding claims, **characterised in that** an antibacterial filter (32) is assigned to the rinsing arrangement (30).

9. The wound treatment assembly according to any of the preceding claims, **characterised in that** the depth of the drainage space is 5 mm or less and/or at least 0.5 mm.

10. The wound treatment assembly according to any of the preceding claims, **characterised by** a covering device, in particular a covering film, that can be fixed, airtight, onto the skin surrounding the wound to be treated and through which the suction tube (20) passes.

## Revendications

1. Agencement de traitement de plaie comportant deux couches de drainage (10, 12) auxquelles est associé un dispositif d'aspiration (20) qui sert à aspirer l'exsudat arrivant entre lesdites couches de drainage (10, 12) et qui est susceptible d'être relié d'un autre côté à un système aspirant, dans lequel le dispositif d'aspiration (20) est associé à un dispositif de rinçage (30) destiné à l'apport d'un fluide entre les couches de drainage (10, 12), **caractérisé en ce qu'**au moins une couche de drainage présente deux éléments en forme de bande pratiquement parallèles l'un à l'autre entre lesquels est constitué un espace de drainage dont la profondeur, dans une direction perpendiculaire aux éléments en forme de bande, assure un effet de capillarité sur les exsudats contenus dans l'espace de drainage.

2. Agencement de traitement de plaie selon la revendication 1, **caractérisé en ce que** le dispositif d'aspiration (20) présente au moins un tuyau d'aspiration comportant une section d'aspiration disposée, avec un embranchement éventuel, entre les couches de drainage (10, 12) ainsi qu'une section de conduite située librement à l'extérieur des couches de drainage (10, 12) et susceptible d'être reliée au système aspirant.

3. Agencement de traitement de plaie selon la revendication 2, **caractérisé en ce qu'**une section d'enveloppe de tuyau de la section d'aspiration présente au moins une et de préférence deux ou trois ouvertures, voire davantage, permettant le guidage de l'exsudat vers la lumière de tuyau entourée de l'enveloppe de tuyau.

4. Agencement de traitement de plaie selon l'une quelconque des revendications précédentes, **caractérisé en ce que** le dispositif de rinçage (30) présente un tuyau de rinçage comportant une section de rinçage débouchant entre les couches de drainage (10, 12) et située de préférence entre les couches de drainage (10, 12), et comportant une section d'apport située librement à l'extérieur des couches de drainage (10, 12).

5. Agencement de traitement de plaie selon l'une quelconque des revendications précédentes, **caractérisé en ce que** dispositif d'aspiration (20) et le dispositif de rinçage (30) présentent un tuyau à plusieurs lumières.

6. Agencement de traitement de plaie selon l'une quelconque des revendications précédentes, **caractérisé par** une couche aspirante située entre les couches de drainage (10, 12) et présentant de préférence de la gaze.

7. Agencement de traitement de plaie selon l'une quelconque des revendications précédentes, **caractérisé en ce que** les couches de drainage (10, 12) sont pratiquement de même superficie et sont jointes, au moins par section, le long de leurs bords circonférentiels, en étant notamment cousues et/ou collées.

8. Agencement de traitement de plaie selon l'une quelconque des revendications précédentes, **caractérisé en ce qu'**un filtre antibactérien (32) est associé au dispositif de rinçage (30).

9. Agencement de traitement de plaie selon l'une quelconque des revendications précédentes, **caractérisé en ce que** la profondeur de l'espace de drainage est de 5 mm ou moins et/ou d'au moins 0,5 mm.

10. Agencement de traitement de plaie selon l'une quelconque des revendications précédentes, **caractérisé par** un dispositif de revêtement, notamment une feuille de revêtement qui est susceptible d'être fixée de manière étanche à l'air sur la peau entourant la plaie à traiter et qui est traversée par le tuyau d'aspiration (20).
